(19) Europäisches Patentamt
European Patent Office
Office européen des brevets

(11) EP 1 940 448 B1

(12) FASCICULE DE BREVET EUROPEEN

(45) Date de publication et mention de la délivrance du brevet:
**06.03.2013 Bulletin 2013/10**

(21) Numéro de dépôt: **06808894.7**

(22) Date de dépôt: **26.09.2006**

(51) Int Cl.:
***A61K 38/18*** *(2006.01)* ***A61K 47/36*** *(2006.01)*
***A61P 17/02*** *(2006.01)*

(86) Numéro de dépôt international:
**PCT/IB2006/002666**

(87) Numéro de publication internationale:
**WO 2007/034320 (29.03.2007 Gazette 2007/13)**

# (54) COMPLEXE POLYMERE AMPHIPHILE-PDGF

PDGF-AMPHIPHILER POLYMER-KOMPLEX

PDGF AMPHIPHILIC POLYMER COMPLEX

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **26.09.2005 FR 0509803**

(43) Date de publication de la demande:
**09.07.2008 Bulletin 2008/28**

(73) Titulaire: **Adocia**
**69003 Lyon (FR)**

(72) Inventeurs:
- **DUBREUCQ, Guy**
**59000 Lille (FR)**
- **DAHRI-CORREIA, Latifa**
**69720 Saint Bonnet de Mure (FR)**
- **CORREIA, José**
**69720 Saint Bonnet de Mure (FR)**
- **DURACHER, David**
**69004 Lyon (FR)**
- **SOULA, Rémi**
**69330 Meyzieu (FR)**
- **SOULA, Olivier**
**69330 Meyzieu (FR)**
- **SOULA, Gérard**
**F-69330 Meyzieu (FR)**

(74) Mandataire: **Tripoz, Inès et al**
**CABINET Inès Tripoz**
**52, rue du Colombier**
**69007 Lyon (FR)**

(56) Documents cités:
**EP-A1- 1 369 136 WO-A-00/76452**
**WO-A-02/058718 WO-A1-00/76562**
**US-A- 5 457 093**

- **CARION O. ET AL.: "Polysaccharide microarray for polysaccharide-PDGF interaction studies." CHEMBIOCHEM, vol. 7, 7 mai 2006 (2006-05-07), pages 817-826, XP002441279**
- **FRANK L ET AL: "Effect of a dextran derivative associated with TGF-beta or FGF-2 on dermal fibroblast behaviour in dermal equivalents" BIOSIS, 2004, XP002396236**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 940 448 B1

## Description

La présente invention concerne de nouveaux complexes de Platelet-Derived Growth Factor (PDGF) associés à des polymères amphiphiles permettant d'améliorer la stabilité physique et chimique in vitro et in vivo de la protéine thérapeutique pour des applications pharmaceutiques.

Les PDGF sont des glycoprotéines d'environ 30000 Daltons, composées de deux chaînes polypeptidiques liées entre elles par deux ponts disulfures. Quatre types de chaînes ont été identifiés A, B, C et D. La protéine native existe sous la forme d'homodimère ou d'hétérodimère de type AB (Oefner C. EMBO J. 11, 3921-2926, 1992).

Le PDGF a été isolé pour la première fois dans les plaquettes. Ce sont des facteurs de croissance libérés lors de la coagulation sanguine, capables de promouvoir la croissance de différents types de cellules (Ross R. et al., Proc. Natl. Acad. Sci. USA, 1974, 71, 1207 ; Kohler N. & Lipton A., Exp. Cell Res., 1974, 87, 297). Il est maintenant connu que le PDGF est produit par un certain nombre de cellules autres que les plaquettes et qu'il est mitogène pour la plupart des cellules dérivées du mésenchyme, i.e. les cellules sanguines, musculaires, osseuses et cartilagineuses, ainsi que les cellules du tissu conjonctif (Raines E.W., in "Biology of Platelet-Derivated Growth Factor", 1993, Westermark, B. et C. Sorg, Ed. Basel, Kerger, p. 74). De nombreux articles tendent également à démontrer que le PDGF issu des macrophages se comporte comme un agent chémotactique et mitogène pour les cellules musculaires lisses, qu'il contribue à l'épaississement myointimal des parois artérielles caractéristique de l'artériosclérose (Ross R. et al., Science, 1990, 248, 1009). Les activités du PDGF incluent en outre et notamment la stimulation de la libération des granules par les monocytes neutrophiles (Tzeng D.Y. et al., Blood, 1985, 66, 179), la facilitation de la synthèse stéroïdienne par les cellules de Leydig (Risbridger G.P., Mol. Cell. Endocrinol., 1993, 97, 125), la stimulation de la phagocytose neutrophile (Wilson E. et al., Proc. Natl. Acad. Sci. USA, 1987, 84, 2213), la modulation de l'expression et de la sécrétion de la thrombospondine (Majak R.A. et al., J. Biol. Chem., 1987, 262, 8821), et la post-régulation du gène ICAM-1 dans les cellules musculaires lisses vasculaires (Morisaki N. et al., Biochem. Biophys. Res. Commun., 1994, 200, 612).

Compte tenu de ces diverses propriétés, l'utilisation de PDGFs recombinants dans le domaine pharmaceutique a déjà été envisagée. L'utilisation du PDGF a notamment été approuvée pour le traitement des ulcères de pied diabétique (Regranex, J&J) et pour la réparation périodontale (GEM 21S, Blomimetic).

La cicatrisation des ulcères, tout comme la cicatrisation cutanée en général, est un phénomène complexe qui nécessite l'intervention coordonnée dans le temps et dans l'espace de nombreux types cellulaires, qui peut se résumer en trois phases : une phase d'inflammation, une phase de prolifération et une phase de remodelage.

Dans la phase inflammatoire, d'environ 7 jours pour une cicatrisation normale, les macrophages tuent les bactéries, débrident les tissus endommagés et régénèrent les tissus. Pour ce faire, les macrophages sécrètent des collagénases, des cytokines et des facteurs de croissance.

Au cours de la phase de prolifération, du 3$^{e}$ jour à la 3$^{e}$ semaine pour une cicatrisation normale, trois événements se succèdent. La plaie se remplit du tissu de granulation, l'angiogénèse se développe et la plaie se couvre de cellules épithéliales. Le tissu de granulation croit des bords vers le centre. Les fibroblastes produisent abondamment du collagène de type III.

Au cours du remodelage, de la 3$^{e}$ semaine à 1 ou même 2 ans, les tissus de granulation maturent, les fibroblastes produisent moins de collagène. Les vaisseaux sanguins formés au cours de la granulation qui sont inutiles sont éliminés par apoptose. Le collagène de type III est remplacé par du collagène de type I qui s'organise selon des lignes de tension et se réticule.

Dans ce processus, le PDGF joue un rôle primordial. Lors de la formation d'une plaie, les plaquettes s'agrègent et libèrent le PDGF. Le PDGF attire les neutrophiles, les macrophages et les fibroblastes sur la plaie et est un puissant mitogène. Les macrophages, les cellules endothéliales synthétisent et sécrètent à leur tour du PDGF. Le PDGF stimule la production de la nouvelle matrice extracellulaire par les fibroblastes, essentiellement les composés non collagéniques tels que les glycosaminoglycanes et les protéines d'adhésion (J. F. Norton et al, Essential practice of surgery, Springer, 2003, chapitre 7, 77-89).

Les plaies chroniques telles que les ulcères de pied diabétique, les ulcères veineux et les ulcères de pression présentent la particularité de cicatriser très lentement et parfois de façon incomplète car le processus de cicatrisation ne se déroule pas normalement (R. Lobmann et al, J. of Diabetes and its complications, 2006, 20, 329-335).

Le processus de cicatrisation est en fait un équilibre délicat entre un processus de destruction nécessaire afin d'éliminer les tissus endommagés et le processus de réparation qui conduit à la formation de nouveaux tissus. Les protéases et les facteurs de croissance jouent un rôle crucial dans ce processus en réglant cet équilibre. Dans le cas des plaies chroniques, cet équilibre est rompu en faveur de la dégradation ce qui explique leur retard à la cicatrisation. Alors qu'il existe différents types de plaies chroniques, biochimiquement elles sont relativement similaires dans le sens où elles sont caractérisées par des phases prolongées d'inflammation conduisant à des niveaux élevés en protéases et diminuant ainsi l'activité des facteurs de croissance (G. Lauer et al. J. Invest. Dermatol. 115 (2000) 12-18). Cette dégradation des facteurs de croissance contribue à une perte globale de tissus associés à ces plaies chroniques ne favorisant pas la cicatrisation (D.R. Yager et al. J. Invest. Dermatol. 107(1996) 743-748).

Il existe actuellement sur le marché un médicament à base de PDGF-BB recombinant humain correspondant à la dénomination commune internationale "becaplermin", vendu sous la dénomination commerciale Regranex®. Ce médicament est indiqué pour le traitement des ulcères des membres inférieurs de diabétiques. Il se présente sous la forme d'un gel à application topique et permet de promouvoir la cicatrisation des ulcères. Il permet en particulier, tout comme le PDGF endogène, de favoriser la prolifération cellulaire et donc la formation de nouveaux tissus.

Ce traitement a une efficacité limitée (Cullen et al. The international journal of biochemistry & Cell Biology 34,1544-1556,2002) même si les études cliniques ont montré des améliorations de la cicatrisation et de la durée nécéssaire à la cicatrisation (Greenhalgh et al., American Journal of pathology,136,1235-1246 1990; Ladin Plastic and Reconstructive Surgery,105,1230-1231 2000 ; Holloway et al. Wounds.5/4,198-206; Mandracchia et al. Clinics in Podiatric Medecine and Surgery,18,189-209 2001; Wieman T.J. American Journal of Surgery,176,74S-79S 1998).

Le produit Regranex qui contient le PDGF-BB, commercialisé par J&J, a démontré son efficacité en augmentant le taux de guérison à 50% des patients traités contre seulement 36% pour les patients qui ont seulement reçu un traitement standard de la plaie. Malgré cette amélioration significative du traitement de l'ulcère de pied diabétique, force est de constater que seulement 50% des patients guérissent après un traitement long et coûteux. Dans les cas de non guérison, les conséquences peuvent être extrêmement graves et conduire dans de nombreux cas à une amputation du membre inférieur. Il faut ajouter que la durée moyenne du traitement est très longue, environ 20 semaines et son application est coûteuse et contraignante à raison d'un nettoyage de la plaie et d'une application de Regranex le matin suivie 12 heures plus tard d'un nettoyage de la plaie. Ces deux interventions nécessitent le plus souvent les soins d'une infimière. De plus le coût moyen d'un traitement d'une durée de vingt semaines est excessivement élevé (de l'ordre de 1400 dollars américains).

L'efficacité partielle peut s'expliquer par une dégradation rapide du PDGF sur la plaie à traiter. Cette dégradation résulte dans le cas d'une plaie chronique d'un état d'inflammation prolongé générant, au niveau de la plaie un environnement hostile au PDGF par stimulation d'une surproduction de protéases.

Alors que le contrôle de la dégradation est nécessaire pour la cicatrisation des plaies, une activité protéolytique excessive est nuisible en conduisant à la dégradation de la matrice extracellulaire (F. Grinnell et al. J. Invest. Dermatol. 106 (1996) 335-341 et C.N. Rao et al. J. Invest. Dermatol. 105 (1995) 572-578) et des molécules ayant un rôle fonctionnel clé comme les facteurs de croissance (V. Falanga et al. J. Derm. Surg. Onc. 18(1992) 604-606 ;D.R. Yager et al. Wound Rep. Reg. 5 (1997) 23-32. et M. Wlaschek et la. Br. J. Dermatol. 137 (1997) 646-647). En effet, les facteurs de croissance comme le PDGF, le TGF$\beta$ ou le bFGF sont des éléments clés dans le processus de cicatrisation par leurs capacités à induire la migration des cellules, la prolifération, la synthèse de protéine, la formation de la matrice et plus généralement en contrôlant le processus de réparation. Cependant ces facteurs de croissance sont des molécules protéiques, et par conséquent sensibles à la dégradation protéolytique. Plusieurs études montrent que la dégradation des facteurs de croissance comme le PDGF est beaucoup plus rapide lorsqu'ils sont mis en contact avec des fluides provenant de plaies chroniques car ils contiennent des concentrations élévées en métalloprotéinases (D.R. Yager et al. J. Invest. Dermatol. 107(1996) 743-748).

Pour le traitement des ulcères veineux Regranex lors d'une étude clinique pilote reportée dans la publication (T.J. Wieman, Wounds, 2003,vol 15, n°8, 257-264) n'a montré qu'une amélioration mineure des traitements actuels à base d'un nettoyage régulier de la plaie avec une thérapie de compression.

Le problème de l'instabilité du PDGF par exemple a été révélé lors de la production de la protéine. Il est connu que le PDGF est particulièrement sensible à la protéolyse post-traductionnelle (Hart et al.,Biochemistry 29 :166-172, 1990 et brevet US serial N°07/557,219) et notamment au niveau de la liaison entre l'acide aminé arginine en position 32 et l'acide aminé thréonine en position 33 de la chaîne mature de la protéine. D'autres sites sont sensibles à la protéolyse comme la liaision entre l'arginine en position 79 et la lysine en position 80 ou encore la liaison entre l'arginine en position 27 et l'arginine en position 28 de la chaîne B du PDGF.

Cette instabilité protéolytique pose un problème majeur dans le cadre de l'obtention de cette protéine qui est produite de façon recombinante chez la levure selon le procédé décrit dans le brevet US N°4,845,075. En effet, le brevet US N°7,084,262 nous apprend que l'analyse et la purification du PDGF-BB, conduit à l'obtention de 21 isoformes résultant de clivages endoprotéolytiques post-traductionnels. Cette grande hétérogénéité structurale a pour conséquence d'entraîner une diminution de 50% de l'activité de la protéine produite par génie génétique par rapport à la protéine intacte de forme mature.

Par ailleurs des résultats cliniques récents de Cardium, d'après un communiqué de presse daté du 14 août 2006 (www.prnewswire.com) sur des ulcères de pied diabétique non guéris après 14 semaines montrent le potentiel que peut offrir un traitement avec le PDGF-BB. La solution proposée par Cardium consiste à introduire le gène d'expression du PDGF-BB dans les cellules de la plaie pour le surexprimer localement. Cette thérapie génique à l'aide d'un adénovecteur a permis de cicatriser près de 80% de ces ulcères du pied diabétique résistants aux traitements courants, sur une cohorte de 15 patients. Cette solution thérapeutique est prometteuse. Mais les développements pharmaceutiques de traitements à base de thérapie génique sont à ce jour encore très hasardeux pour des raisons de sécurité liées à l'emploi de vecteurs viraux de type adénovirus.

Il y a donc un besoin et une possibilité d'amélioration les traitements actuels de l'ulcère de pied diabétique avec le PDGF.

Dans le cas du traitement de l'ulcère de pied diabétique, l'objectif ultime est triple :

➢ accélérer la guérison
➢ augmenter le taux de guérison
➢ simplifier le protocole du traitement

Il y a aussi le cas des ulcères veineux et des ulcères de pression, causes de douleurs importantes et de complications médicales très graves.

Le problème à résoudre est donc pour l'essentiel une protection du PDGF sur la plaie chronique.

Diverses solutions ont été proposées.

Le brevet US 5,905,142 décrit un moyen de remédier à ces problèmes de protéolyse concernant le PDGF en générant des mutants de la protéine ayant une résistance accrue vis-à-vis des attaques protéolytiques en substituant ou en supprimant un ou plusieurs acides aminés lysine ou arginine proches des sites de clivage potentiels. Cette stratégie pour rendre la protéine plus résistante aux protéases n'est pas satisfaisante. Cette modification génétique du PDGF peut entraîner des modifications de l'activité biologique avec des affinités différentes vis-à-vis de ces différents récepteurs ce qui peut aussi induire des problèmes toxicologiques. De plus une telle modification du PDGF nécessite un nouveau développement pharmaceutique, extrèmement coûteux et hasardeux.

Dans les années 1970 lorsque cette protéine a été abondamment étudiée, Il s'est révélé que la purification était extrêmement délicate car le PDGF est « une protéine très collante » de part ses propriétés cationiques et hydrophobes (Heldin,C.H. EMBO J. 11 :4251-4259 ,1992 ; Raines and Ross,J.Biol.Chem. 257(9):5154-5160,1982 ; Antoniades, PNAS 78 :7314, 1981 ; Deuel et al. J. Biol. Chem. 256:8896, 1981). Le PDGF est en effet une protéine fortement cationique dont le point isoélectrique est compris entre 9,8 et 10,5. D'autres auteurs confirment ce comportement comme Wei et al. (Journal of controlled release 112 :103-110, 2006) qui expliquent que le PDGF s'adsorbe aisément sur des surfaces du contenant dans lequel il se trouve en solution. Les auteurs solutionnent le problème en ajoutant dans le mélange soit de la BSA à 0,1%, soit un mélange 0,1%BSA/Tween 20. Ces solutions résolvent en grande partie le problème puisque jusqu'à 95% de la protéine se retrouve en solution. Mais ces solutions ne sont pas satisfaisantes d'un point de vue pharmaceutique étant donné l'origine animal de la BSA et les risques liés à l'encéphalopathie spongiforme bovine.

Une autre solution proposée par les mêmes auteurs, consiste à ajouter un surfactant anionique plus puissant (SDS) qui permet de maintenir le PDGF en solution. Malheureusement, le SDS induit également une dénaturation partielle de la protéine conduisant à une perte de la bioactivité. Cette solution n'est donc pas satisfaisante pour stabiliser la protéine.

Dans le brevet W093/08825, les inventeurs ont mis en évidence que le PDGF purifié présente une forte instabilité lorsqu'il est formulé sous la forme d'un gel pour une application topique. Ils donnent pour exemple l'incompatibilité du PDGF avec un certain nombre de produits utilisés classiquement pour formuler des produits pharmaceutiques comme la méthylcellulose ou l'hydroxypropylcellulose ainsi que certains conservateurs conventionnels comme l'alcool benzylique. Les auteurs posent le problème en expliquant qu'il existe un besoin de formuler le PDGF sous la forme d'un gel pour administration topique tout en ayant une bonne stabilité long terme. Les mêmes auteurs montrent que le PDGF en solution se dégrade par un processus de déamidation à pH neutre et que la protéine est plus stable à un pH légèrement acide. Les auteurs montrent qu'en combinant plusieurs paramètres, un polymère ne présentant pas d'interactions avec la protéine, un tampon à pH légèrement acide permettant de limiter la réaction de déamidation et un conservateur neutre vis-à-vis de la protéine, Il est possible de formuler le PDGF afin d'obtenir une formulation stable d'un point de vue pharmaceutique.

Les auteurs montrent qu'il est possible d'obtenir une formulation stable au stockage par ajout d'un polymère ne présentant pas d'interactions avec la protéine à conditions de maintenir la formulation à pH legèrement acide afin d'éviter les réactions de dégradation par déamidation de la protéine. Cette solution n'est toutefois pas satisfaisante car elle ne permet pas de protéger le facteur de croissance des dégradations protéolytiques in vivo au pH physiologique.

Dans le brevet WO97/12601 qui décrit des formulations de PDGF sous la forme de gel, les auteurs expliquent que le dérivé de la cellulose qu'ils utilisent, est capable de stabiliser les facteurs de croissance envers une éventuelle perte d'activité lors du stockage. Pour cela ils se basent sur les résultats obtenus précédemment sur l'EGF dans le brevet US 4,717,717. Cependant ils expliquent également que la stabilité du gel de cellulose contenant du PDGF peut être grandement améliorée en ajoutant à la formulation une espèce chimique chargée tel que des acides aminés chargés ou des ions métalliques. La encore, cette solution permet de stabiliser les facteurs de croissance dans le formulation lors du stockage du produit mais ne permet pas une stabilisation de ces facteurs de croissance vis-à-vis des protéases présentes au niveau des plaies chroniques au pH physiologique.

Il existe donc un intérêt thérapeutique à stabiliser et protéger les facteurs de croissance, notamment le PDGF,

afin d'augmenter son efficacité dans le cadre d'un traitement des plaies chroniques et plus particulièrement celui des plaies d'ulcères de pied diabétique. L'invention concerne donc la stabilisation du PDGF vis-à-vis des dégradations chimiques ou physiques pouvant intervenir au pH physiologique in vitro et in vivo par la mise au point d'un complexe entre un polymère amphiphile et un PDGF.

L'invention concerne donc la formation d'un complexe entre un polymère amphiphile et un PDGF (polymère amphiphile-PDGF), ce complexe apportant une stabilisation chimique et physique à la protéine vis-à-vis des dégradations à pH physiologique *in vitro* et *in vivo.*

La présente invention concerne donc un complexe polymère amphiphile-PDGF, stable physiquement et chimiquement, soluble dans l'eau, caractérisé en ce que :

- les polymères amphiphiles sont constitués d'un squelette polymérique hydrophile fonctionnalisé par des substituants hydrophobes et des groupements hydrophiles selon la formule générale suivante.

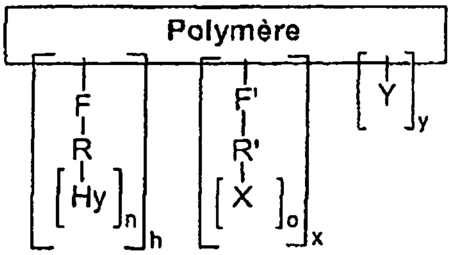

DP = m unités monomère

le polymère étant choisi dans le groupe constitué par les dextranes et les celluloses,

● R, R' étant une liaison ou une chaîne comprenant entre 1 et 18 carbones, éventuellement branchée et/ou insaturée comprenant un ou plusieurs hétéroatomes, tels que O, N ou/et S, R et R' étant identiques ou différents entre eux

● F, F' étant un ester, un thioester, un amide, un carbonate, un carbamate, un éther, un thioéther, une amine, F et F' étant identiques ou différents entre eux

● X étant un groupement hydrophile : choisi dans le groupe constitué par :

o un carboxylate

o un sulfate

o un sulfonate

o un phosphate

o un phosphonate

● Y étant un groupement hydrophile : choisi dans le groupe constitué par :

o un sulfate

o un sulfonate

o un phosphate

o un phosphonate

● Hy étant un groupement hydrophobe : choisi dans le groupe constitué par

o un alkyle linéaire ou ramifié en C8 à C30, éventuellement insaturé et/ou contenant un ou plusieurs hétéroatomes, tels que O, N ou S.

o un alkylaryl ou un arylalkyle linéaire ou ramifié en C8 à C18, éventuellement insaturé et/ou contenant éventuellement un hétéroatome

o un polycycle en C8 à C30 éventuellement insaturé.

n et o sont compris entre 1 et 3,
h représente la fraction molaire de motif hydrophobe par rapport à une unité monomérique comprise entre 0.01 et 0.5
x représente la fraction molaire de groupements hydrophiles par rapport à une unité monomérique, comprise entre 0 et 2.0.
y représente la fraction molaire de groupements hydrophiles par rapport à une unité monomérique, comprise entre 0 et 0.5.

- le PDGF est choisi dans le groupe des PDGFs (Platelet-Derivated Growth Factors).

Elle concerne un complexe caractérisé en ce que le PDGF est choisi dans le groupe constitué par les PDGFs recombinants humains comportant deux chaînes B (rhPDGF-BB).
Elle concerne un complexe caractérisé en ce que le PDGF est le PDGF-BB.
Les substituants des polymères amphiphiles sont répartis de façon contrôlée ou statistique. Parmi les polymères ayant une répartition contrôlée des substituants, on peut citer, par exemple, les copolymères à blocs et les copolymères alternés.
Copolymère statistique

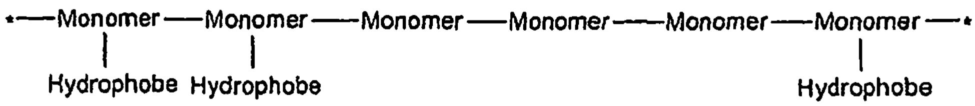


Copolymère à blocs

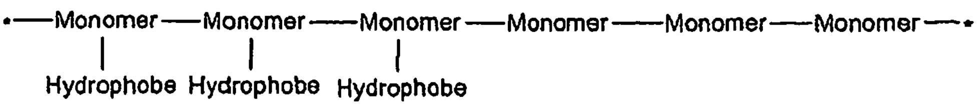


Copolymère alterné

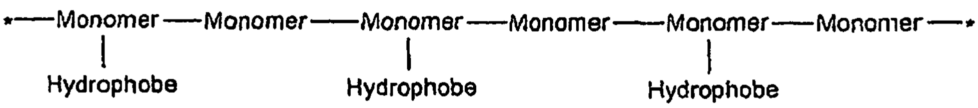


Ainsi dans un mode de réalisation, l'invention concerne également un complexe polymère amphiphile-PDGF caractérisé en ce que le polymère est choisi parmi les polymères dont les substituants sont répartis de façon statistique.
Dans un mode de réalisation, l'invention concerne également un complexe polymère amphiphile-PDGF caractérisé en ce que le polymère est choisi parmi parmi les polysaccharides.
Dans un mode de réalisation, les polysaccharides sont choisis dans le groupe constitué par les hyaluronanes, les alginates, les chitosans, les galacturonanes, la chondroitin-sulfate, les dextrans, les celluloses.
Le groupe des celluloses est constitué des celluloses fonctionnalisées par des acides comme la carboxyméthylcellulose.
Le groupe des dextrans est constitué des dextrans fonctionnalisés par des acides comme la carboxyméthyldextran.

Mode de réalisation non revendiqué

Dans un mode de réalisation également décrit, le polysaccharide est un dérivé de dextran soluble répondant à la formule (I) suivante :

$DMC_aB_bSu_c$ (I)

dans laquelle :

- D représente une chaîne polysaccharidique, de préférence constituée par des enchaînements d'unités glucosidiques,

- MC représente des groupes méthylcarboxylique,
- B représente des groupes N-benzylméthylénecarboxamide,
- Su représente des groupes sulfates (sulfatation des fonctions hydroxyle libres, portées par les unités glucosidiques),
- a, b et c représentent le degré de substitution (ds), respectivement des groupements MC, B et Su avec

  i) a strictement supérieur à 0 ;
  ii) b est tel que :

    . soit b est supérieur ou égal à 0,3 et c est compris entre 0,1 et 0,5 ;

    . soit b est strictement Inférieur à 0,3 et c répond à l'équation (1) suivante :

$$c \geq 8{,}5\, b^2 - 5{,}41\, b + 0{,}86 \qquad (1).$$

Ces dérivés de dextran de formule (I), ainsi que leur procédé de préparation sont décrits de façon plus générale dans la demande de brevet WO 99/29734. Ces dérivés de dextran de formule (I) sont trivialement appelés DMCBSu et sont considérés comme étant des copolymères constitués de sous-unités R-OH et R-OX, X pouvant être un groupement méthylcarboxylique (MC), benzylamide (B) ou sulfate (Su). Ainsi, un dextran méthylcarboxylique (DMC) avec un degré de substitution (ds) de 0,6 en groupements méthylcarboxyliques contient 0,6 groupement substitué (R-MC) et 2,4 de groupements hydroxyles (R-OH), par unité glucosidique.

Dans un mode de réalisation, D présente une masse molaire comprise entre 1 000 et 2 000 000 Da, et dans un mode de réalisation, inférieure à 70 000 Da.

Dans un mode de réalisation, les dérivés de dextran sont choisis parmi les composés de formule (I) dans lesquels b est supérieur ou égal à 0,35.

Dans ce cas, et selon un mode de réalisation, les dérivés de dextran sont choisis parmi les composés de formule (I) dans lesquels a est compris entre 0,5 et 0,8, et c est compris entre 0,1 et 0,5.

Dans un mode de réalisation, les polysaccharides sont choisis dans le groupe constitué par les hyaluronanes, les alginates, les chitosans.

Ces différents polysaccharides peuvent être représentés comme suit :

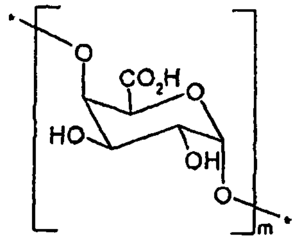

Galacturonane

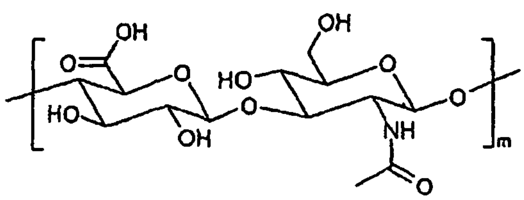

Hyaluronane

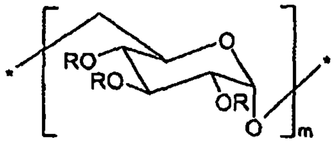

R = H, Dextran
R = $CH_2COOH$ ou H, Carboxymethyl Dextran

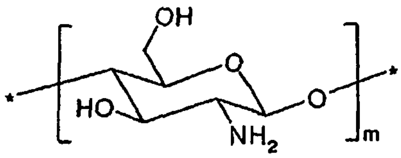

Chitosan

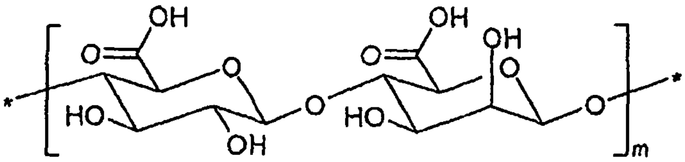

Alginate

Le polysaccharide peut avoir un degré de polymérisation moyen m compris entre 10 et 10000.

Dans un mode de réalisation, il a un degré de polymérisation moyen m compris entre 10 et 5000.

Dans un autre mode de réalisation, il a un degré de polymérisation moyen m compris entre 10 et 500.

Dans un mode de réalisation, l'invention concerne également un complexe polymère amphiphile-PDGF caractérisé en ce que le groupe hydrophobe Hy est choisi dans le groupe constitué par les acides gras, les alcools gras, les amines grasses, les amines benzyliques, les dérivés du cholestérol et les phénols.

Dans un mode de réalisation, le dérivé du cholestérol est l'acide cholique.

Dans un autre mode de réalisation, le phénol est l'alpha-tocophérol.

Dans un mode de réalisation, le complexe polymère amphiphile-PDGF selon l'invention est réversible.

Les polymères utilisés sont synthétisés selon les techniques connues de l'Homme de l'art ou achetés auprès de fournisseurs comme par exemple Sigma-Aldrich, NOF Corp. ou CarboMer Inc.

Les PDGF sont choisis parmi les PDGF recombinants humains, obtenus selon les techniques connues de l'Homme de l'art ou achetés auprès de fournisseurs comme par exemple auprès des sociétés Gentaur (USA) ou Research Diagnostic Inc. (USA).

La mise en évidence de la stabilisation à la fois chimique et physique peut être effectuée notamment par la mise en oeuvre des tests suivants :

- un test de mise en évidence du complexe polymère amphiphile-PDGF selon l'invention, réalisé par électrophorèse sur gel (Gel Mobility Shift Assay)
- un test de ralentissement de la dégradation enzymatique d'un complexe polymère amphiphile-PDGF selon l'invention réalisé par mise en contact avec une protéase
- un test de stabilisation physique d'un complexe polymère amphiphile-PDGF selon l'invention à pH physiologique réalisé par SDS-Page.

L'invention concerne également un complexe polymère amphiphile-PDGF caractérisé en ce qu'il satisfait aux tests de mise en évidence de la stabilisation chimique et physique, à savoir un test de mise en évidence du complexe (Gel Mobility Shift Assay), le test de ralentissement de la dégradation enzymatique par mise en contact avec une protéase et le test de stabilisation physique à pH physiologique réalisé par SDS-Page.

Le test de mise en évidence du complexe réalisé par Gel Mobility Shift Assay basé sur le déplacement d'ions sous l'effet d'un champ électrique. Les complexes anioniques migrent vers l'anode et les complexes cationiques se déplacent vers la cathode. Après migration, les protéines sont transférées par capillarité sur membrane de PVDF et révélées par un anticorps spécifique de la protéine reconnu par un deuxième anticorps couplé à la peroxidase. La protéine seule ne migre pas ou peu, la protéine complexée au polymère amphiphile migre vers l'anode ou la cathode en fonction de la charge globale du complexe.

Le test de ralentissement de la dégradation enzymatique est basé sur la vérification de l'intégrité de la protéine après mise en contact du complexe polymère amphiphile-PDGF selon l'invention, avec une protéase. Une solution d'une protéase (trypsine, chymotrypsine...) est ajoutée à la solution de complexe et une cinétique est réalisée. La réaction est arrêtée par ajout d'un inhibiteur spécifique de l'enzyme (indole, benzamidine). L'intégrité de la protéine est ensuite

analysée par électrophorèse sur gel de polyacrylamide (SDS-Page).

Le test de stabilisation physique d'un PDGF est basé sur la vérification de l'intégrité de la protéine par comparaison d'une solution du complexe polymère amphiphile-PDGF selon l'invention à une solution de PDGF seule à pH 7.4 en terme de concentration en protéine dans la solution. Ces deux solutions sont placées sur un banc d'agitation pendant 48h à température ambiante puis centrifugées. La concentration en PDGF dans chacune des solutions est évaluée par SDS-Page.

Le complexe polymère amphiphile-PDGF selon l'invention est formé par la mise en solution aqueuse d'un PDGF et d'un polymère amphiphile à pH physiologique sans utiliser de solvant organique susceptible de dénaturer la protéine. La formation du complexe polymère amphiphile-PDGF est spontanée et n'implique pas de liaison covalente entre le PDGF et le polymère amphiphile. Cette association se fait par des liaisons faibles qui sont essentiellement des interactions hydrophobes et des interactions ioniques.

L'invention concerne également le procédé de préparation du complexe polymère amphiphile-PDGF selon l'invention, caractérisé en ce que l'on met en contact en solution à pH physiologique un PDGF et un polymère amphiphile.

D'autres tests peuvent être mis en place pour parfaire la mise en évidence de la formation du complexe polymère amphiphile-PDGF selon l'invention.

- un test de maintien de la structure tertiaire du PDGF déterminé par dichroïsme circulaire
- un test de stabilité d'un PDGF dans le complexe polymère amphiphile-PDGF selon l'invention à pH physiologique sous stress: Le stress peut être un mode d'agitation particulier, la présence de sels, etc.

L'invention concerne également un complexe polymère amphiphile-PDGF caractérisé en ce que le ratio PDGF/polymère amphiphile est compris entre 1/5 et 1/5000.

Dans un mode de réalisation, il est compris entre 1/100 et 1/5000.

Dans un autre mode de réalisation, il est compris entre 1/300 et 1/700.

L'invention concerne également une composition thérapeutique caractérisée en ce qu'elle comprend un complexe polymère amphiphile-PDGF selon l'invention.

On entend par composition thérapeutique une composition utilisable en médecine humaine ou vétérinaire.

La composition pharmaceutique selon l'invention est de préférence une composition à application topique pouvant se présenter sous la forme d'un gel, de crème, d'un sprays ou d'une pâte, ou d'un patch.

La nature des excipients susceptibles d'être formulés avec le complexe polymère amphiphile-PDGF selon l'invention est choisie en fonction de sa forme de présentation selon les connaissances générales du galéniste.

Ainsi, lorsque la composition selon l'invention est sous la forme d'un gel, celui-ci est par exemple un gel réalisé à partir de polymères tels que les carboxyméthylcelluloses (CMC) les polymères vinyliques, les copolymères de type PEO-PPO, les polysaccharides, les PEO, les acrylamides ou les dérivés d'acrylamides.

D'autres excipients peuvent etre utilisés dans cette invention afin d'ajuster les paramètres de la formulation comme un tampon pour ajuster le pH, un agent permettant d'ajuster l'isotonicité, des conservateurs comme le parahydroxybenzoate de méthyle, le parahydroxybenzoate de propyle, le m-crésol, ou le phénol ou encore un agent antioxydant comme le chlorhydrate de L-lysine.

Selon l'invention, la composition thérapeutique est caractérisée en ce qu'elle permet une administration d'environ 100 $\mu$g par ml de PDGF.

La présente invention concerne également l'utilisation d'un complexe polymère amphiphile-PDGF selon l'invention pour la préparation d'une composition thérapeutique à action cicatrisante destinée au traitement des ulcères par voie topique.

Elle concerne également une méthode de traitement thérapeutique à usage humain ou vétérinaire caractérisée en ce qu'elle consiste à administrer au site de traitement une composition thérapeutique comprenant le complexe polymère amphiphile-PDGF selon l'invention.

## EXEMPLES

### Exemple de référence 1 : Complexe PDGF-BB/DMCBSu ne correspondant pas à un objet revendiqué

#### Synthèse du carboxyméthyl dextran sulfaté modifié par la benzylamine (DMCBSu)

Le polymère amphiphile est synthétisé à partir d'un carboxyméthyl dextran ayant un degré de substitution en carboxyméthyl par unité saccharidique de 1.0 et une masse molaire moyenne de 40000 g/mol. La benzylamine est greffée sur les acides de ce polymère selon une méthode classique de couplage dans l'eau en présence d'un carbodiimide hydrosoluble. Le degré de substitution en benzylamine par unité saccharidique est de 0.4, déterminé par RMN $^1$H. Ce polymère est ensuite sulfaté par un complexe SO3/pyridine, Le degré de substitution en sulfate par unité saccharidique

est de 0.3.

Préparation du complexe PDGF-BB/DMCBSu

10 µl d'une solution de PDGF-BB à 0,1 mg/ml est ajoutée à 90 µl d'une solution de DMCBSu à 50 mg/ml. Les solutions de PDGF-BB et de DMCBSu sont tamponnées à pH 7.4 et 300 mOsm. Cette solution est mise sous agitation douce deux heures à température ambiante puis stockée à 4°C.

Mise en évidence de la formation d'un complexe PDGF-BB/DMCBSu

10 µl de la solution du complexe PDGF-BB/ DMCBSu décrite ci-dessus est déposée sur un gel d'agarose. La migration des composés s'effectue sous l'effet d'un champ électrique (200mA - 4 heures). Après migration, le PDGF-BB est transféré sur une membrane de PVDF durant une nuit, puis révélé par immunoblotting avec des anticorps de chèvre anti-PDGF-BB sur lesquels se fixeront des anticorps secondaires anti-IgG de chèvre couplés à la peroxidase HRP révélée par un substrat (5-bromo-4-chloro-3-indolyl phosphate/nitro blue tetrazolium).

Le complexe PDGF-BB/DMCBSu migre vers l'anode. Sa charge négative s'explique par une composition beaucoup plus riche en DMCBSu qu'en PDGF-BB. Le contrôle, constitué uniquement de PDGF-BB, ne migre pas.

Mise en évidence de la stabilité de la solution du complexe PDGF- DMCBSu

10 µl d'une solution de PDGF-BB à 0,01 mg/ml à pH 7.4 et 10 µl de la solution du complexe PDGF-BB /DMCBSu à pH 7.4 décrite ci-dessus sont placées sur un banc d'agitation pendant 48h à température ambiante. Après centrifugation, la concentration en PDGF-BB dans chacune des solutions est évaluée par SDS-Page. Il apparaît que la concentration en PDGF-BB en solution dans le cas du complexe PDGF-BB /DMCBSu n'a pas changé alors que celle de la solution de PDGF-BB seule a diminué.

Mise en évidence de la protection du PDGF-BB contre la trypsine dans ce complexe PDGF-BB/ DMCBSu

10 µl de la solution du complexe BMP-2/DMCTrpOMe décrite ci-dessus sont versés dans 90 µl d'une solution de trypsine à 10 ng/ml à 37°C.

Par un prélèvement de 10 µl, la concentration en PDGF-BB est mesurée toutes les 30 minutes par Elisa après avoir arrêté la réaction enzymatique par l'ajout de 10 µl d'une solution d'indole à 10 µg/ml.

Cette cinétique révèle que le PDGF-BB seul est totalement dégradé en 1 h30 alors qu'il ne l'est pas dans le complexe PDGF-BB/ DMCBSu.

Validation de l'activité biologique du complexe PDGF-BB/ DMCBSu

Une culture primaire des fibroblastes de derme humain (Human Dermal Fibroblast adult (HDFa) est réalisée à une température de 37°C dans du milieu αMEM avec 10% de sérum de veau foetal (SVF) et 1 % de pénicilline-streptomycine dans une atmosphère saturée en humidité et enrichie en CO2 (5%). Le milieu est renouvelé tous les 4 jours. Une dilution de la suspension cellulaire dans le milieu de culture a ensuite été réalisée pour ensemencer les boîtes de culture à une densité de 5.000 cellules/puits pour des plaques de 96 puits (société Nunc).

Pour chaque lot de cellules, l'effet stabilisant du PDGF-BB par le complexe à différentes concentrations, a été vérifié par incorporation de thymidine tritiée (5000 cellules/puits dans 100 µl). Après 24 heures de sevrage, les fibroblastes sont stimulés par addition de PDGF-BB, à différentes concentrations allant de 0,1 à 100 ng/ml, en présence ou non du polymère amphiphile, à une concentration de 1 µg/ml. L'incorporation de la thymidine tritiée est effectuée 18 heures après la stimulation par le PDGF-BB en présence ou non du complexe, par addition d'une solution à 50 µCi/ml, soit 0,5 µCi/puits. La radioactivité est récupérée dans des fioles de comptage, les puits ont été rincés par 100 µl de NaOH 100 mM et la radioactivité est comptée après addition de 1 ml de liquide de scintillation (Zinsser Analytic), sur un compteur automatique.

Les résultats obtenus sont représentés sur la figure 1 annexée sur laquelle la quantité de thymidine tritiée incorporée par les fibroblastes (en Dpm x 103), est exprimée en fonction de la concentration en PDGF-BB en µg/ml.

La courbe en trait plein représente les résultats du complexe selon l'invention à une concentration de 1 µg/ml en dérivé de dextran et la courbe en traits pointillés les résultats en absence du dérivé de dextran.

L'ED50 correspond à la concentration en PDGF-BB pour avoir 50 % de prolifération des fibroblastes humain. Le ratio R est le rapport des ED50 calculé comme suit :

$$R = ED50\ (PDGF\text{-}BB) / ED50\ (PDGF\text{-}BB + DMCBSu)$$

Ces résultats montrent que lorsque le PDGF-BB est utilisé seul, il est nécessaire d'en employer 6 μg/ml pour obtenir 50 % de prolifération, alors que lorsque le PDGF-BB est complexé par un dérivé de dextran de formule (I), il suffit de 2 μg/ml pour atteindre 50 % de prolifération. Le ratio R, dans ce cas, est égal à 3.

Exemple 2 : *Complexe PDGF-BB/DMCTrpOMe*

Synthèse du carboxyméthyl dextran modifié par l'ester méthylique du *tryptophane (DMCTrpOMe)*

Le polymère amphiphile est synthétisé à partir d'un carboxyméthyl dextran ayant un degré de substitution en carboxyméthyl par unité saccharidique de 1.0 et une masse molaire moyenne de 40000 g/mol. L'ester méthylique du tryptophane est greffé sur les acides de ce polymère selon une méthode classique de couplage dans l'eau en présence d'un carbodiimide hydrosoluble. Le degré de substitution en tryptophane par unité saccharidique est de 0.3 déterminé par RMN 1H.

Préparation du complexe PDGF-BB/DMCTrpOMe

10 μl d'une solution de PDGF-BB à 0,1 mg/ml est ajoutée à 90 μl d'une solution de DMCTrpOMe à 50 mg/ml. Les solutions de PDGF-BB et de DMCTrpOMe sont tamponnées à pH 7.4 et 300 mOsm. Cette solution est mise sous agitation douce deux heures à température ambiante puis stockée à 4°C.

Mise en évidence de la formation d'un complexe PDGF-BB/DMCTrpOMe

10 μl de la solution du complexe PDGF-BB/DMCTrpOMe décrite cl-dessus est déposée sur un gel d'agarose pour une électrophorèse sur gel avec une révélation immunologique. La migration des composés s'effectue sous l'effet d'un champ électrique (200mA - 4 heures). Après migration, le PDGF-BB est transféré sur une membrane de PVDF durant une nuit, puis révélé par immunoblotting avec des anticorps de chèvre anti-PDGF-BB sur lesquels se fixeront des anticorps secondaires anti-IgG de chèvre couplés à la peroxidase révélée par un substrat (5-bromo-4-chloro-3-indolyl phosphate/nitro blue tetrazolium).

Le complexe PDGF-BB/DMCTrpOMe migre vers l'anode. Sa charge négative s'explique par une composition beaucoup plus riche en DMCTrpOMe qu'en PDGF-BB. Le contrôle, constitué uniquement de PDGF-BB, ne migre pas.

Mise en évidence de la stabilité du complexe PDGF-BB/DMCTrpOMe

10 μl d'une solution de PDGF-BB à 0,01 mg/ml à pH 7.4 et 10 μl de la solution du complexe PDGF-BB/DMCTrpOMe à pH 7.4 décrite ci-dessus sont placées sur un banc d'agitation pendant 48h à température ambiante. La concentration en PDGF-BB dans chacune des solutions est évaluée par SDS-Page. Il apparaît que la concentration en PDGF-BB en solution dans le cas du complexe PDGF-BB/DMCTrpOMe n'a pas changé alors que celle de la solution de PDGF-BB seule a diminué.

Mise en évidence de la protection du PDGF-BB contre la trypsine dans ce complexe PDGF-BB/DMCTrpOMe

10 μl de la solution du complexe PDGF-BB/DMCTrpOMe décrite ci-dessus est incubée avec 90μL une solution de Trypsine à 10 ng/mL à 37°C. Par un prélèvement de 10 μl toutes les 30 minutes, l'intégrité structurale du PDGF-BB est évaluée par électrophorèse sur gel de polyacrylamide (SDS-Page) après avoir arrêté la réaction enzymatique par l'ajout de 10 μl d'une solution d'indole à 10 μg/ml

Cette cinétique révèle que le PDGF-BB seul est totalement dégradé en 1h30 alors qu'il ne l'est pas dans le complexe PDGF-BB/DMCTrpOMe.

*Exemple de référence 3 : Complexe PDGF-BB/CMCBSu* ne correspondant pas à un objet revendiqué

Synthèse de la carboxyméthyl cellulose sulfaté modifié par la benzylamine (CMCBSu)

Le polymère amphiphile est synthétisé à partir d'une carboxyméthyl cellulose ayant un degré de substitution en carboxyméthyl par unité saccharidique de 1.2 et une masse molaire moyenne de 30000 g/mol. La benzylamine est

greffée sur les acides de ce polymère selon une méthode classique de couplage dans l'eau en présence d'une carbodiimide hydrosoluble. Le degré de substitution en benzylamine par unité saccharidique est de 0.2 déterminé par RMN $^1$H. La sulfatation est réalisée en présence d'un complexe SO3-Pyridine, le degré de substitution en sulfate est de 0.30

Préparation du complexe PDGF-BB/CMCBSu

10 µl d'une solution de PDGF-BB à 0,1 mg/ml est ajoutée à 90 µl d'une solution de CMCB à 50 mg/ml. Les solutions de PDGF-BB et de CMCBSu sont tamponnées à pH 7.4 et 300 mOsm. Cette solution est mise sous agitation douce deux heures à température ambiante puis stockée à 4°C.

Mise en évidence de la formation d'un complexe PDGF-BB/CMCBSu

10 µl de la solution du complexe PDGF-BB/CMCBSu décrite ci-dessus est déposée sur un gel d'agarose pour une électrophorèse sur gel avec une révélation immunologique. La migration des composés s'effectue sous l'effet d'un champ électrique (200mA - 4 heures). Après migration, le PDGF-BB est transféré sur une membrane de PVDF durant une nuit, puis révélé par immunoblotting avec des anticorps de chèvre anti-PDGF-BB sur lesquels se fixeront des anticorps secondaires anti-IgG de chèvre couplés à la peroxidase HRP révélée par un substrat (5-bromo-4-chloro-3-indolyl phosphate/nitro blue tetrazolium).

Le complexe PDGF-BB/CMCBSu migre vers l'anode. Sa charge négative s'explique par une composition beaucoup plus riche en CMCBSu qu'en PDGF-BB. Le contrôle, constitué uniquement de PDGF-BB, ne migre pas.

Mise en évidence de la stabilité du complexe PDGF-BB/CMCBSu

10 µl d'une solution de PDGF-BB à 0,01 mg/ml à pH 7.4 et 10 µl de la solution du complexe PDGF-BB / CMCBSu à pH 7.4 décrite ci-dessus sont placés sur un banc d'agitation pendant 48h à température ambiante. Après la centrifugation, la concentration en PDGF-BB dans chacune des solutions est évaluée par SDS-Page. Il apparaît que la concentration en PDGF-BB en solution dans le cas du complexe PDGF-BB/CMCBSu n'a pas changé alors que celle de la solution de PDGF-BB seule a diminué.

Mise en évidence de la protection du PDGF-BB contre la trypsine dans ce complexe PDGF-BB/CMCBSu

10 µl de la solution du complexe PDGF-BB/CMCBSu décrite ci-dessus est incubée avec 90µL une solution de Trypsine à 10 ng/mL à 37°C. Par un prélèvement de 10 µl toutes les 30 minutes, l'intégrité structurale du PDGF-BB est évaluée par électrophorèse sur gel de polyacrylamide (SDS-Page) après avoir arrêté la réaction enzymatique par l'ajout de 10 µl d'une solution d'indole à 10 µg/ml.

Cette cinétique révèle que le PDGF-BB seul est totalement dégradé en 1 h30 alors qu'il ne l'est pas dans le complexe PDGF-BB/DMCBsu.

*Contre-exemple 1: complexe PDGF-BB/CMCB*

Synthèse de la carboxyméthyl cellulose modifié par la benzylamine (CMCB)

Le polymère amphiphile est synthétisé à partir d'une carboxyméthyl cellulose ayant un degré de substitution en carboxyméthyl par unité saccharidique de 1.2 et une masse molaire moyenne de 30000 g/mol. La benzylamine est greffée sur les acides de ce polymère selon une méthode classique de couplage dans l'eau en présence d'une carbodiimide hydrosoluble. Le degré de substitution en benzylamine par unité saccharidique est de 0.2 déterminé par RMN $^1$H.

Préparation du complexe PDGF-BB/CMCB

10 µl d'une solution de PDGF-BB à 0,1 mg/ml est ajoutée à 90 µl d'une solution de CMCB à 50 mg/ml. Les solutions de PDGF-BB et de CMCB sont tamponnées à pH 7.4 et 300 mOsm. Cette solution est mise sous agitation douce deux heures à température ambiante puis stockée à 4°C.

Mise en évidence de la non formation de complexe PDGF-BB/CMCB

10 µl de la solution du complexe PDGF-BB/CMCB décrite ci-dessus est déposée sur un gel d'agarose pour une électrophorèse sur gel avec une révélation immunologique. La migration des composés s'effectue sous l'effet d'un champ électrique (200mA - 4 heures). Après migration, le PDGF-BB est transféré sur une membrane de PVDF durant

une nuit, puis révélé par immunoblotting avec des anticorps de chèvre anti-PDGF-BB sur lesquels se fixeront des anticorps secondaires anti-IgG de chèvre couplés à la peroxidase HRP révélée par un substrat (5-bromo-4-chloro-3-indolyl phosphate/nitro blue tetrazolium).

La révélation ne montre pas de migration du PDGF-BB seul avec le polymère amphiphile. Il n'y a pas eu formation de complexe entre le CMCB et le PDGF-BB.

## Revendications

1. Complexe polymère amphiphile-PDGF, stable physiquement et chimiquement, soluble dans l'eau, **caractérisé en ce que** :

    les polymères amphiphiles sont constitués d'un squelette polymérique hydrophile fonctionnalisé par des substituants hydrophobes et des groupements hydrophiles selon la formule générale suivante,

    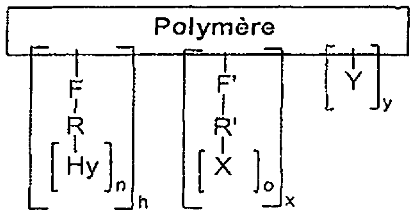

    DP = m unités monomère

    ● le polymère étant choisi dans le groupe constitué par les dextrans et les celluloses.
    ● R, R' étant une liaison ou une chaîne comprenant entre 1 et 18 carbones, éventuellement branchée et/ou insaturée comprenant un ou plusieurs hétéroatomes, tels que 0, N ou/et S,
    R et R' étant identiques ou différents entre eux,
    ● F, F' étant un ester, un thioester, un amide, un carbonate, un carbamate, un éther, un thioéther, une amine,
    F et F' étant identiques ou différents entre eux
    ● X étant un groupement hydrophile choisi dans le groupe constitué par :

    o un carboxylate
    o un sulfate
    o un sulfonate
    o un phosphate
    o un phosphonate

    ● Y étant un groupement hydrophile choisi dans le groupe constitué de :

    o un sulfate
    o u n sulfonate
    o un phosphate
    o un phosphonate

    ●Hy étant un groupement hydrophobe choisi dans le groupe constitué de :

    o un alkyle linéaire ou ramifié en C8 à C30, éventuellement insaturé et/ou contenant un ou plusieurs hétéroatomes, tels que O, N ou S.
    o un alkylaryl ou un arylalkyle linéaire ou ramifié en C8 à C18, éventuellement insaturé et/ou contenant éventuellement un hétéroatome
    o un polycycle en C8 à C30 éventuellement insaturé.

    n et o sont compris entre 1 et 3,
    h représente la fraction molaire de motif hydrophobe par rapport à une unité monomérique comprise entre 0.01 et 0.5

x représente la fraction molaire de groupements hydrophiles par rapport à une unité monomérique, comprise entre 0 et 2.0.
y représente la fraction molaire de groupements hydrophiles par rapport à une unité monomérique, comprise entre 0 et 0.5.

- le PDGF est choisi dans le groupe des PDGFs (Platelet-Derivated Growth Factors).

**2.** Complexe selon la revendication 1, **caractérisé en ce que** le PDGF est choisi dans le groupe constitué par les PDGFs recombinants humains comportant deux chaînes B (rhPDGF-BB).

**3.** Complexe selon la revendication 1, **caractérisé en ce que** le polymère est choisi parmi les polymères dont les substituants sont répartis de façon statistique.

**4.** Complexe selon la revendication 1, **caractérisé en ce que** le groupe hydrophobe Hy est choisi dans le groupe constitué par les acides gras, les alcools gras, les amines grasses, les amines benzyliques, les dérivés du cholestérol et les phénols.

**5.** Complexe selon la revendication 1, **caractérisé en ce que** le groupe des celluloses est constitué des celluloses fonctionnalisées par des acides comme la carboxyméthylcellulose.

**6.** Complexe selon la revendication 1, **caractérisé en ce que** le groupe des dextrans est constitué des dextrans fonctionnalisés par des acides comme le carboxyméthyldextran.

**7.** Complexe selon la revendication 1, **caractérisé en ce que** le polymère est le carboxyméthyl dextran modifié par l'ester méthylique du tryptophane.

**8.** Complexe selon l'une quelconque des revendications précédentes, **caractérisé en ce que** la formation du complexe polymère amphiphile-PDGF est réversible.

**9.** Complexe selon l'une quelconque des revendications précédentes, **caractérisé en ce qu'**il satisfait aux tests de mise en évidence de la stabilisation chimique et physique.

**10.** Complexe selon la revendication 9, **caractérisé en ce que** les tests de mise en évidence de la stabilisation chimique et physique sont choisis dans le groupe constitué du test de mise en évidence du complexe (Gel Mobility Shift Assay), du test de ralentissement de la dégradation enzymatique par mise en contact avec une protéase et du test de stabilisation physique à pH physiologique réalisé par SDS-Page.

**11.** Complexe selon l'une quelconque des revendications précédentes, **caractérisé en ce que** le ratio PDGF/polymère amphiphile est compris entre 1/5 et 1/5000.

**12.** Complexe selon l'une quelconque des revendications précédentes, **caractérisé en ce que** le ratio PDGF/polymère amphiphile est compris entre 1/100 et 1/5000.

**13.** Complexe l'une quelconque des revendications précédentes, **caractérisé en ce que** le ratio PDGF/polymère amphiphile est compris entre 1/300 et 1/700.

**14.** Procédé de préparation du complexe polymère amphiphile-PDGF selon l'une quelconque des revendications précédentes, **caractérisé en ce que** l'on prépare ce complexe polymère/PDGF-BB en milieux aqueux et en absence de solvant organique susceptible de dénaturer la protéine.

**15.** Composition thérapeutique **caractérisée en ce qu'**elle comprend un complexe polymère amphiphile-PDGF selon l'une quelconque des revendications 1 à 13.

**16.** Composition thérapeutique selon la revendication 15, **caractérisée en ce qu'**elle permet une administration de 100 μg par ml de PDGF,

**17.** Complexe selon l'une quelconque des revendications 1 à 13 pour l'utilisation dans le traitement des ulcères par voie topique.

## Claims

1. Physically and chemically stable, water-soluble, amphiphilic polymer-PDGF complex, **characterized in that**:

   the amphiphilic polymers consist of a hydrophilic polymeric backbone functionalized with hydrophobic substituents and hydrophilic groups according to the following general formula:

   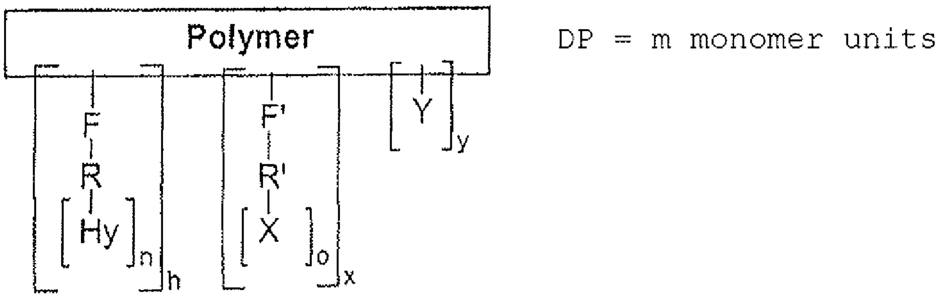

   - the polymer being chosen from the group consisting of dextrans and celluloses,
   - R and R' being a bond or a chain comprising between 1 and 18 carbons, optionally branched and/or unsaturated comprising one or more heteroatoms, such as 0, N and/or S,
   R and R' being identical to or different from one another,
   - F and F' being an ester, a thioester, an amide, a carbonate, a carbamate, an ether, a thioether, an amine, F and F' being identical to or different from one another,
   - X being a hydrophilic group chosen from the group consisting of:

     - a carboxylate
     - a sulphate
     - a sulphonate
     - a phosphate
     - a phosphonate,

   - Y being a hydrophilic group chosen from the group consisting of:

     - a sulphate
     - a sulphonate
     - a phosphate
     - a phosphonate,

   - Hy being a hydrophobic group chosen from the group consisting of:

     - a $C_8$ to $C_{30}$ linear or branched alkyl, optionally unsaturated and/or containing one or more heteroatoms, such as O, N or S,
     - a $C_8$ to $C_{18}$ linear or branched alkylaryl or arylalkyl, optionally unsaturated and/or optionally containing a heteroatom,
     - an optionally unsaturated $C_8$ to $C_{30}$ polycycle, n and O are between 1 and 3,

   h represents the molar fraction of hydrophobic unit relative to a monomeric unit of between 0.01 and 0.5,
   x represents the molar fraction of hydrophilic groups
   relative to a monomeric unit, of between 0 and 2.0,
   y represents the molar fraction of hydrophilic groups relative to a monomeric unit, of between 0 and 0.5;

   - the PDGF is chosen from the group of PDGFs (platelet-derived growth factors).

2. Complex according to Claim 1, **characterized in that** the PDGF is chosen from the group consisting of human recombinant PDGFs containing two B chains (rhPDGF-BB).

3. Complex according to Claim 1, **characterized in that** the polymer is chosen from polymers in which the substituents are distributed randomly.

4. Complex according to Claim 1, **characterized in that** the hydrophobic group Hy is chosen from the group consisting of fatty acids, fatty alcohols, fatty amines, benzyl amines, cholesterol derivatives and phenols.

5. Complex according to Claim 1, **characterized in that** the group of celluloses consists of celluloses functionalized with acids, such as carboxymethylcellulose.

6. Complex according to Claim 1, **characterized in that** the group of dextrans consists of dextrans functionalized with acids, such as carboxymethyl dextran.

7. Complex according to Claim 1, **characterized in that** the polymer is carboxymethyl dextran modified with tryptophan methyl ester.

8. Complex according to any one of the preceding claims, **characterized in that** the formation of the amphiphilic polymer-PDGF complex is reversible.

9. Complex according to any one of the preceding claims, **characterized in that** it passes the tests for demonstration of the chemical and physical stabilization.

10. Complex according to Claim 9, **characterized in that** the tests for demonstration of the chemical and physical stabilization are chosen from the group consisting of the test for demonstration of the complex (Gel Mobility Shift Assay), the test for slowing down of the enzymatic degradation by bringing into contact with a protease and the test for physical stabilization at physiological pH carried out by SDS-Page.

11. Complex according to any one of the preceding claims, **characterized in that** the PDGF/amphiphilic polymer ratio is between 1/5 and 1/5000.

12. Complex according to any one of the preceding claims, **characterized in that** the PDGF/amphiphilic polymer ratio is between 1/100 and 1/5000.

13. Complex according to any one of the preceding claims, **characterized in that** the PDGF/amphiphilic polymer ratio is between 1/300 and 1/700.

14. Method of preparing the amphiphilic polymer/PDGF complex according to any one of the preceding claims, **characterized in that** this polymer/PDGF-BB complex is prepared in aqueous media and in the absence of any organic solvent that may denature the protein.

15. Therapeutic composition, **characterized in that** it comprises an amphiphilic polymer-PDGF complex according to any one of Claims 1 to 13.

16. Therapeutic composition according to Claim 15, **characterized in that** it allows an administration of 100 $\mu$g per ml of PDGF.

17. Complex according to any one of Claims 1 to 13, for use in the treatment of ulcers by topical application.

## Patentansprüche

1. Physikalisch und chemisch stabiler, wasserlöslicher Komplex aus amphiphilem Polymer und PDGF, **dadurch gekennzeichnet, dass**
die amphiphilen Polymere aus einem hydrophilen Polymerrückgrat bestehen, das mit hydrophoben Substituenten und hydrophilen Gruppen gemäß der folgenden allegemeinen Formel substituiert ist:

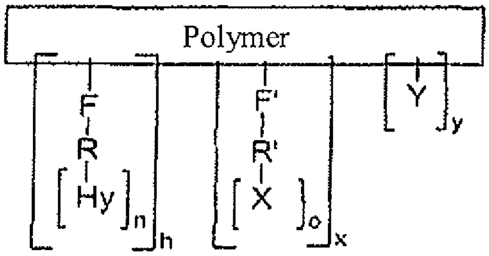

DP = m Monomereinheiten

● das Polymer ausgewählt ist aus der Gruppe umfassend Dextrane und Cellulosen,

● R, R' eine Bindung oder eine Kette umfassend zwischen 1 und 18 Kohlenstoffen ist, optional verzweigt und/oder ungesättigt, umfassend ein oder mehrere Heteroatome wie beispielsweise O, N und/oder S,
R und R' identisch oder voneinander verschieden sind,

● F, F' ein Ester, ein Thioester, ein Amid, ein Carbonat, ein Carbamat, ein Ether, Thioether, ein Amin ist,
F und F' identisch oder voneinander verschieden sind

● X eine hydrophile Gruppe ist, die ausgewählt ist aus der Gruppe umfassend:

o ein Carboxylat
o ein Sulfat
o ein Sulfonat
o ein Phosphat
o ein Phosphonat

● Y eine hydrophile Gruppe ist, die ausgewählt ist aus der Gruppe umfassend:

o ein Sulfat
o ein Sulfonat
o ein Phosphat
o ein Phosphonat

● Hy eine hydrophobe Gruppe ist, die ausgewählt ist aus der Gruppe umfassend:
○ ein lineares oder verzweigtes C8 bis C30-Alkyl, optional ungesättigt und/oder ein oder mehrere Heteroatome wie beispielsweise O, N oder S enthaltend
○ ein lineares oder verzweigtes C8 bis C18-Alkylaryl oder -Arylalkyl, optional ungesättigt und/oder optional enthaltend ein Heteroatom
○ einen optional ungesättigten C8 bis C30-Polyzyklus n und o zwischen 1 und 3 enthalten sind,
h den Molenbruch von hydrophobem Strukturelement zu einer Monomereinheit darstellt, enthalten zwischen 0,01 und 0,5
x den Molenbruch von hydrophilen Gruppen zu einer Monomereinheit darstellt, enthalten zwischen 0 und 2,0
y den Molenbruch von hydrophilen Gruppen zu einer Monomereinheit darstellt, enthalten zwischen 0 und 0,5

- der PDGF ausgewählt ist aus der Gruppe der PDGFs (Plättchen-gewonnener Wachstumsfaktoren).

2. Komplex nach Anspruch 1, **dadurch gekennzeichnet, dass** der PDGF ausgewählt ist aus der Gruppe umfassend menschliche rekombinante PDGFs enthaltend zwei B-Ketten (rhPDGF-BB).

3. Komplex nach Anspruch 1, **dadurch gekennzeichnet, dass** das Polymer ausgewählt ist aus Polymeren, deren Substituenten statistisch verteilt sind.

4. Komplex nach Anspruch 1, **dadurch gekennzeichnet, dass** die hydrophobe Gruppe Hy ausgewählt ist aus der Gruppe umfassend Fettsäuren, Fettalkohole, Fettsäureamine, Benzylamine, Cholesterolderivate und Phenole.

5. Komplex nach Anspruch 1, **dadurch gekennzeichnet, dass** die Gruppe der Cellulosen aus Cellulosen besteht, die mit Säuren funktionalisiert sind, wie beispielsweise Carboxymethylcellulose.

6. Komplex nach Anspruch 1, **dadurch gekennzeichnet, dass** die Gruppe der Dextrane ausgebildet ist aus Dextranen, die mit Säuren funktionalisiert sind, wie beispielsweise Carboxymethyldextran.

7. Komplex nach Anspruch 1, **dadurch gekennzeichnet, dass** das Polymer Carboxymethyldextran ist, das mit dem Methylester von Tryptophan modifiziert ist.

8. Komplex nach einem der vorangehenden Ansprüche, **dadurch gekennzeichnet, dass** die Ausbildung des Komplexes aus amphiphilem Polymer und PDGF reversibel ist.

9. Komplex nach einem der vorangehenden Ansprüche, **dadurch gekennzeichnet, dass** er Tests zum Nachweisen der chemischen und physikalischen Stabilität genügt.

10. Komplex nach Anspruch 9, **dadurch gekennzeichnet, dass** die Tests zum Nachweisen der chemischen und physikalischen Stabilität ausgewählt sind aus der Gruppe umfassend einen Test zum Nachweisen des Komplexes (Gelmobilitäts-Shift-Test), Test zum Verlangsamen des enzymatischen Abbaus durch Kontaktieren mit einer Protease und Test auf physikalische Stabilisierung bei physiologischem pH, durchgeführt durch SDS-PAGE.

11. Komplex nach einem der vorangehenden Ansprüche, **dadurch gekennzeichnet, dass** das Verhältnis von PDGF/amphiphiles Polymer zwischen 1/5 und 1/5000 beträgt.

12. Komplex nach einem der vorangehenden Ansprüche, **dadurch gekennzeichnet, dass** das Verhältnis PDGF/amphiphiles Polymer zwischen 1/100 und 1/5000 beträgt.

13. Komplex nach einem der vorangehenden Ansprüche, **dadurch gekennzeichnet, dass** das Verhältnis PDGF/amphiphiles Polymer zwischen 1/300 und 1/700 beträgt.

14. Verfahren zum Herstellen des Komplexes aus amphiphilem Polymer und PDGF nach einem der vorangehenden Ansprüche, **dadurch gekennzeichnet, dass** der Komplex Polymer/PDGF-BB in wässrigem Milieu und in Abwesenheit von organischem Lösemittel, das das Protein denaturieren kann, hergestellt wird.

15. Therapeutische Zusammensetzung, **dadurch gekennzeichnet, dass** sie einen Komplex aus amphiphilem Polymer und PDGF nach einem der Ansprüche 1 bis 13 umfasst.

16. Therapeutische Zusammensetzung nach Anspruch 15, **dadurch gekennzeichnet, dass** sie eine Verabreichung von 100 μg pro ml von PDGF erlaubt.

17. Komplex nach einem der vorangehenden Ansprüche 1 bis 13 zur Verwendung bei der Behandlung von Ulcera auf topischem Wege.

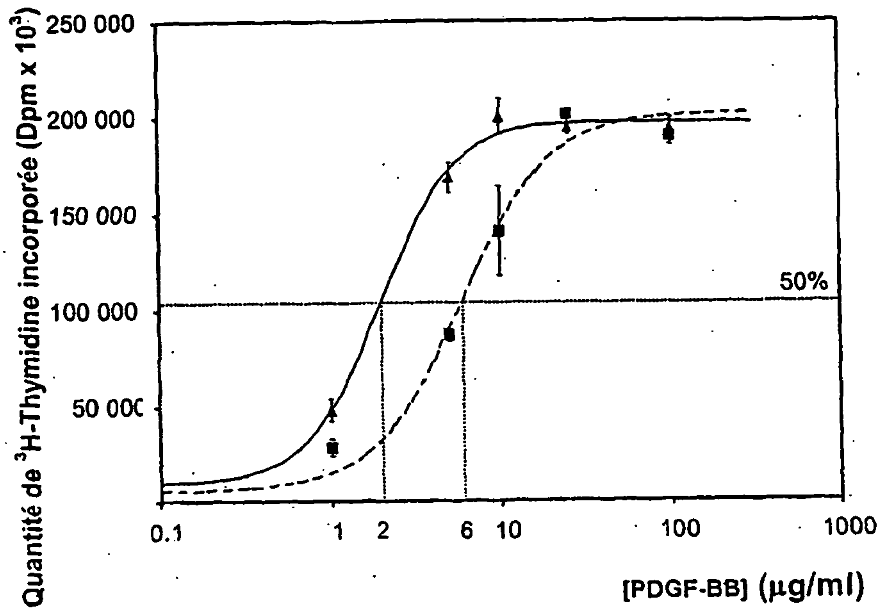
Quantité de $^3$H-Thymidine incorporée (Dpm x $10^3$)
250 000
200 000
150 000
100 000
50 000
50%
0.1
1
2
6
10
100
1000
[PDGF-BB] (μg/ml)

FIGURE 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 07557219 B
- US 4845075 A
- US 7084262 B
- US 5905142 A
- WO 9308825 A
- WO 9712601 A
- US 4717717 A
- WO 9929734 A


### Littérature non-brevet citée dans la description


- **OEFNER C.** *EMBO J.,* 1992, vol. 11, 3921-2926
- **ROSS R. et al.** *Proc. Natl. Acad. Sci. USA,* 1974, vol. 71, 1207
- **KOHLER N. ; LIPTON A.** *Exp. Cell Res.,* 1974, vol. 87, 297
- **RAINES E.W.** Biology of Platelet-Derivated Growth Factor. 1993, 74
- **ROSS R. et al.** *Science,* 1990, vol. 248, 1009
- **TZENG D.Y. et al.** *Blood,* 1985, vol. 66, 179
- **RISBRIDGER G.P.** *Mol. Cell. Endocrinol.,* 1993, vol. 97, 125
- **WILSON E. et al.** *Proc. Natl. Acad. Sci. USA,* 1987, vol. 84, 2213
- **MAJAK R.A. et al.** *J. Biol. Chem.,* 1987, vol. 262, 8821
- **MORISAKI N. et al.** *Biochem. Biophys. Res. Commun.,* 1994, vol. 200, 612
- **J. F. NORTON et al.** Essential practice of surgery. Springer, 2003, 77-89
- **R. LOBMANN et al.** *J. of Diabetes and its complications,* 2006, vol. 20, 329-335
- **G. LAUER et al.** *J. Invest. Dermatol.,* 2000, vol. 115, 12-18
- **D.R. YAGER et al.** *J. Invest. Dermatol.,* 1996, vol. 107, 743-748
- **CULLEN et al.** *The international journal of biochemistry & Cell Biology,* 2002, vol. 34, 1544-1556
- **GREENHALGH et al.** *American Journal of pathology,* 1990, vol. 136, 1235-1246
- *Ladin Plastic and Reconstructive Surgery,* 2000, vol. 105, 1230-1231
- **HOLLOWAY et al.** *Wounds,* vol. 5/4, 198-206
- **MANDRACCHIA et al.** *Clinics in Podiatric Medecine and Surgery,* 2001, vol. 18, 189-209
- **WIEMAN T.J.** *American Journal of Surgery,* 1998, vol. 176, 74S-79S
- **F. GRINNELL et al.** *J. Invest. Dermatol.,* 1996, vol. 106, 335-341
- **C.N. RAO et al.** *J. Invest. Dermatol.,* 1995, vol. 105, 572-578
- **V. FALANGA et al.** *J. Derm. Surg. Onc.,* 1992, vol. 18, 604-606
- **D.R. YAGER et al.** *Wound Rep. Reg.,* 1997, vol. 5, 23-32
- **M. WLASCHEK.** *Br. J. Dermatol.,* 1997, vol. 137, 646-647
- **T.J. WIEMAN.** *Wounds,* 2003, vol. 15 (8), 257-264
- **HART et al.** *Biochemistry,* 1990, vol. 29, 166-172
- **HELDIN,C.H.** *EMBO J.,* 1992, vol. 11, 4251-4259
- **RAINES ; ROSS.** *J.Biol.Chem.,* 1982, vol. 257 (9), 5154-5160
- **ANTONIADES.** *PNAS,* 1981, vol. 78, 7314
- **DEUEL et al.** *J. Biol. Chem.,* 1981, vol. 256, 8896
- **WEI et al.** *Journal of controlled release,* 2006, vol. 112, 103-110